# EUROPEAN PATENT SPECIFICATION

(11) **EP 0 375 845 B1**
(45) Date of publication and mention of the grant of the patent: **05.01.1994**
(21) Application number: 89117486.4
(22) Date of filing: 21.09.1989
(51) Int. Cl.: A61M 16/12

(54) **A high-safety mixer device useful with an apparatus for measuring and monitoring medical fluids**
Hochsicherheitsmischer zur Verwendung in einem Mess- und Steuergerät für medizinische Flüssigkeiten
Mélangeur haute sécurité destiné à un appareil de mesure et de monitorage de fluides médicaux

(30) Priority: 28.12.1988 IT 2312688
(43) Date of publication of application: 04.07.1990
(73) Proprietor: FLOW-METER S.p.A., I-24020 Levate Bergamo (IT)
(72) Inventor: Paratico, Roberto, I-24020 Levate Bergamo (IT)
(74) Representative: Checcacci, Giorgio

(56) References cited:
- FR-A- 2 203 481
- GB-A- 2 001 249
- GB-A- 2 148 721
- US-A- 3 896 837

## Description

This invention relates to a high-safety mixer device useful with an apparatus for measuring and monitoring at least two medical fluids to be mixed together which are fed into the apparatus from respective feed lines.

The invention is particularly, but not exclusively, concerned with a safety device adapted to be incorporated to a flowmeter for anaesthesia treatment, and the ensuing description will make reference to just that field of application for convenience of illustration.

Medical apparatus are known which are operative to measure and control the volumetric flow rates of a pair of fluids in a gaseous state, such as oxygen and nitrous oxide through a flowmeter for anaesthetic treatment.

Nitrous oxide is an anaesthetic gas, and great care is to be exerted to prevent it from being administered to a patient in a pure state, and accordingly, it is always delivered admixed to oxygen in a volumetric flow rate ratio which is preset by a specialized anaesthesia operator.

To meet this demand, medical apparatus are widely employed at present of the kind specified above which, while basically serving their purposes, still have a serious drawback.

The respective oxygen and nitrous oxide streams are fed into the apparatus from respective lines equipped with control valves. Thus, the anaesthetist will set the flow rate of each fluid for delivery of a predetermined ratio mixture thereof.

Consequently, the drawback exists that the delivery of a mixture containing at least a substantial proportion of oxygen is entirely entrusted to the anaesthetist's own skill.

A known apparatus providing for automatically cutting of the delivery of nitrous oxide, on an outage of oxygen pressure, is disclosed in GB-A-2148721.

This document discloses a mixer device useful with an apparatus for measuring and monitoring at least two medical fluids to be mixed together, the fluids being fed into the apparatus from respective feed lines. Such mixer device comprises a pressure switch, having a membrane-type pressure chamber connected in the feed line of one fluid, and a pressure control valve connected in the feed line of the other fluid. The opening of the valve is controlled directly by the pressure force acting on the membrane.

Another similar solution is disclosed in FR-A-2203481 whereby the membrane-type pressure chamber substantially corresponds to a piston sealed by an O-ring and slidably guided in a pressure chamber.

Both the above solutions, while basically serving their purposes, don't provide for further increase in the oxygen flow rate at the occurrence of a higher pressure force.

The technical problem that underlies this invention is to provide a mixer device useful with an apparatus for measuring and monitoring medical fluids, which has such structural and performance characteristics as to cut off in a most reliable manner the delivery of one of the fluids on a pressure outage of the other fluid, while ensuring delivery of a mixture which contains at least a predetermined amount of one of said fluids and obviating, therefore, the above-noted drawbacks with which the prior art is beset.

This problem is solved by a mixer device as specified in the annexed claim 1.

The features and advantages of the device according to this invention will be apparent from the following detailed description of an embodiment thereof, given by way of example and not of limitation with reference to the accompanying drawings.

In the drawings:

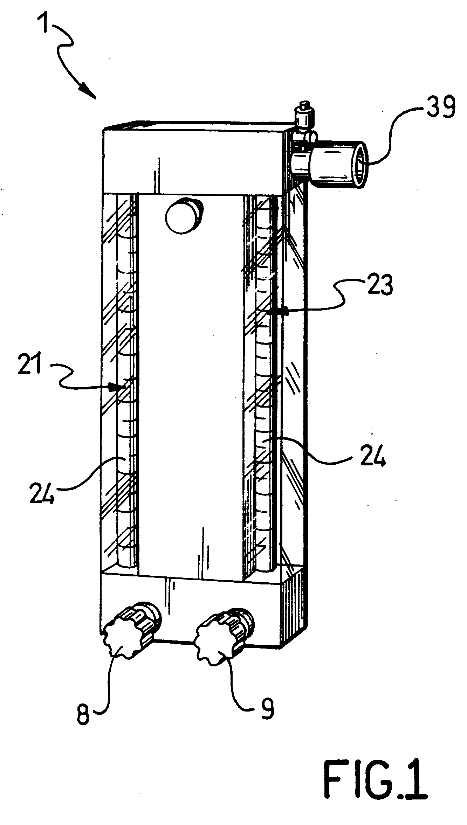

Figure 1 is a perspective view of an apparatus for measuring and monitoring medical fluids incorporating a mixer device according to the invention; and

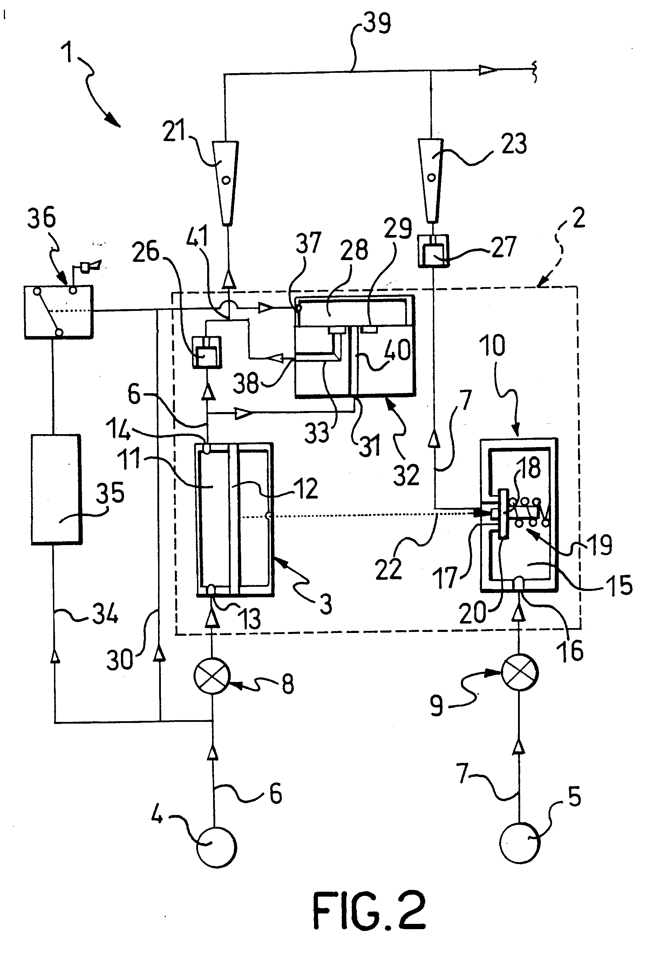

Figure 2 is a schematic view of the mixer device incorporated to the apparatus of Figure 1.

With reference to the drawing views, generally and schematically shown at 1 is an apparatus for measuring and monitoring medical fluid flows.

The apparatus 1 is a flowmeter for anaesthesia treatment, that is an apparatus operative to measure and control the flow rates by volume of a pair of gaseous-state fluids. Specifically, such fluids are oxygen and nitrous oxide, the latter being an anaesthetic gas.

Advantageously, the apparatus 1 incorporates a high-safety mixer device 2 intended, according to the invention, for setting with the utmost precision the mixture ratio of said gases.

Such gases are fed into the apparatus 1 from respective lines 6 and 7.

The device 2 is connected, on the one side, to an oxygen supply source 4 via the line 6, and on the other side, to a nitrous oxide supply source 5 via the line 7.

Connected in each of the lines 6 and 7, upstream of the device 2, are respective fluid flow rate control valves 8 and 9 which are operated manually.

Advantageously, the device 2 includes a pressure switch 3 comprising a pressure chamber 11 housing an elastic membrane 12 on its interior.

The chamber 11 has an inlet 13 and an outlet 14, and is connected in the oxygen feed line 6.

The device 2 further includes a pressure control valve 10 which comprises a chamber 15 having an inlet 16 and an outlet 17 and being connected in the nitrous oxide feed line 7.

The valve 10 has a plunger 18 and respective shutter 20 which is guided, against the bias of a calibrated spring means 19, inside the chamber 15 for movement toward and away from the chamber outlet 17.

The plunger 18 is driven by the membrane 12 and controlled directly by the membrane movements via a link schematically indicated by an arrow 22.

The respective outlets 14 and 17 of the chambers 11 and 15 are connected to the inlet ends of corresponding flowmeters 21 and 23 of the float valve type, each having a calibrated scale 24. Said outlets are each connected to their respective flowmeters by a corresponding gauged injector as indicated at 26 and 27.

The device 2 further includes an additional pressure chamber 28 formed by a solid body 32 accomodating an elastic membrane 29 therein.

A through-going bore 40 is formed in the solid portion of the body 32 which provides an inlet 31 for the chamber 28 connected directly to the outlet 14 of the chamber 11.

The membrane 29 extends almost across the bottom of the chamber 28 and separates the port formed by the bore 40 from a further inlet 37 of the chamber 28. That inlet 37 is connected directly to the oxygen line 6, upstream of the knob 8, by a bypass line 30.

In parallel with the bypass line 30 is an additional safety bypass connection 34 in which a storage tank 35 is connected. A sound warning device 36 is also provided on the connection 34, downstream from the tank 35, to warn of any outage in the oxygen pressure.

The solid portion of the body 32 also has an additional through-going bore 33 extending substantially at right angles, which communicates the bottom of the chamber 28 with an outlet 38 connected to the inlet of the flowmeter 21 via a connection node 41 located downstream from the injector 26 to thereby bypass the injector 26.

The respective outlets of the flowmeters 21 and 23 are connected to a single gas mixture delivery line 39.

The operation of the mixer device according to the invention will be described next.

The oxygen from the supply source 4 is fed into the chamber 11 through the control knob 8. The gauged injector 26 is effective to create a load loss in the circuit, and the bore 40 is kept closed by the membrane 29 the pressure force whereof is provided by the pressure of the oxygen admitted upstream of the knob 8 via the bypass line 30.

Under this condition, the pressure inside the chamber 11 may rise to apply a significant pressure force to the membrane 12.

On the other side, the nitrous oxide from the supply 5, as admitted through the knob 9, is shut off by the shutter 20 of the control valve 10.

The oxygen pressure exerted on the membrane 12 will drive the plunger 18 of that valve 3 to allow the nitrous oxide to leak past the outlet 17 of the chamber 15 to the gauged injector 27, and hence, the flowmeter 23.

The higher is the oxygen flow rate through the chamber 11 the greater is the pressure force acting on the membrane 12, and consequently the extent of the valve 10 opening, thereby the flow rate of the nitrous oxide is set.

The stroke length of the membrane 12 is limited by a stop, and the nitrous oxide highest flow rate is determined by this stroke end.

On the oxygen pressure reaching such a level that no further increase in flow rate is allowed through the gauged injector 26, the oxygen will begin to exert a higher pressure force on the membrane 29 in the chamber 28 through the bore 40. This causes the membrane 29 to flex upwards and allows the oxygen to flow, through the bore 33 and the outlet 38 of the chamber 28, into the flowmeter 21 until a maximum flow rate value is achieved as permitted by the passage cross-section of the valve 8.

It may be appreciated from the foregoing discussion that the device of this invention has a major advantage in that it enables a mixture to be delivered which contains at all times a proportion of oxygen, thus preventing a patient from being administered pure nitrous oxide in the event of any setting error by the anaesthetist.

Furthermore, the device of this invention provides for the delivery of nitrous oxide to be automatically cut off in the event of an outage in the oxygen pressure, to thus make the anaesthesia apparatus whereto it is incorporated highly reliable and safe to use.

## Claims

1. A mixer device (2) useful with a flowmetering apparatus (1) mixing together at least two medical fluids fed from respective feed lines (6, 7), said mixer device comprising:
- a pressure switch (3) having a membrane-type (12) pressure chamber (11) connected in the feed line (6) of one fluid,
- a pressure control valve (10), connected in the feed line of the other fluid, comprising a chamber (15) having an inlet (16) and an outlet (17), a plunger (18) and a respective shutter (20) being guided toward and away from said outlet (17) against the bias of a calibrated spring means (19), said plunger (18) being connected to said membrane and controlled by the pressure force acting on said membrane (12), characterized in that it comprises,
- a gauged injector (26) connected to the outlet side of said pressure chamber (11) and to the inlet side of a flowmeter (21) delivering said one fluid,
- a second pressure chamber (28) provided with an elastic membrane (29) and having a first inlet (37), connected to the feed line (6) of said one fluid as to bypass said pressure switch (3), and a second inlet (31) connected to the outlet side (14) of said pressure switch (3) and separed from the first inlet (37) by said membrane (29),
- said second chamber (28) also having an outlet (38) provided on the same side of the second inlet (31) and connected to the flowmeter (21) downstream from said injector (26).

2. Mixer device according to claim 1, characterized in that it comprises a further bypass connection (34) between the feed line (6) and the first inlet (37) of said second chamber (28), said connection (34) including a storage tank (35).

3. Mixer device according to claim 2, characterized in that a sound warning device (36) is also provided on said connection (34) downstream from the tank (35), for warning any outage in said one fluid pressure.

4. Mixer device according to claim 1, characterized in that said pressure chamber (28) is formed in a solid body (32) accomodating also the elastic membrane (29), a through-going bore (40) being formed in the solid portion of the body (32) providing the first inlet (31) of said second chamber (28).

5. Mixer device according to claim 1, characterized in that said pressure control valve (10) has an outlet (17) connected to a respective inlet of a second flowmeter (23) by a corresponding gauged injector (27).

## Patentansprüche

1. Mischvorrichtung (2) für ein Durchflußmessgerät (1) zum Zusammenmischen von mindestens zwei medizinischen Fluiden, welche jeweils von Zuleitungen (6,7) eingespeist werden, mit
- einem Druckschalter (3) mit einer membranartigen (12) Druckkammer (11), die in der Zuleitung (6) des einen Fluids angeschlossen ist,
- einem Drucksteuerventil (10), das in der Zuleitung des anderen Fluids angeschlossen ist, mit einer Kammer (15), die einen Einlaß (16) und einen Auslaß (17), einen Plunger (18) und eine entsprechende Verschlußklappe (20) hat, die gegen die Vorspannung einer geeichten Federvorrichtung (19) in Richtung zu dem und weg von dem Auslaß (17) geführt sind, wobei der Plunger (18) mit der Membran verbunden und durch die auf die Membran (12) wirkende Druckkraft gesteuert ist,
**gekennzeichnet** durch
- einen kalibrierten Injektor (26), der mit der Auslaßseite der Druckkammer (11) und der Einlaßseite eines Durchflußmessers (21), der das eine Fluid abgibt, verbunden ist,
- eine zweite Druckkammer (28), die eine elastische Membran (29) aufweist und einen ersten Einlaß (37) hat, der mit der Zuleitung (6) des einen Fluids verbunden ist, um den Druckschalter (3) zu umgehen, und einen zweiten Einlaß (31) hat, der mit der Auslaßseite (14) des Druckschalters (3) verbunden und von dem ersten Einlaß (37) durch die Membran (29) getrennt ist,
- wobei die zweite Kammer (28) auch einen Auslaß (38) hat, der auf derselben Seite wie der Zweite Einlaß (31) vorgesehen ist und mit dem Durchflußmesser (21) stromabwärts von dem Injektor (26) verbunden ist.

2. Mischvorrichtung nach Anspruch (1), dadurch gekennzeichnet, daß ferner eine Bypass-Verbindung (34) zwischen der Zuleitung (6) und dem ersten Einlaß (37) der zweiten Kammer (28) vorgesehen ist, wobei die Verbindung (34) einen Speichertank (35) umfaßt.

3. Mischvorrichtung nach Anspruch (2), dadurch gekennzeichnet, daß eine Ton-Warnvorrichtung (36) an der Verbindung (34) stromabwärts von dem Tank (35) vorgesehen ist, um bei einem Ausfall des einen Fluiddruckes zu warnen.

4. Mischvorrichtung nach Anspruch 1, dadurch gekennzeichnet, daß die Druckkammer (28) in einem Vollkörper (32) ausgebildet ist, der auch die elastische Membran (29) aufnimmt, wobei eine Durchgangsbohrung (40) in dem festen Abschnitt des Körpers (32) ausgebildet ist, um den ersten Einlaß (31) der zweiten Kammer (28) vorzusehen.

5. Mischvorrichtung nach Anspruch 1, dadurch gekennzeichnet, daß das Drucksteuerventil (10) einen Auslaß (17) aufweist, der mit einem entsprechenden Einlaß eines zweiten Durchflußmessers (23) über einen zugeordneten kalibrierten Injektor (27) verbunden ist.

## Revendications

1. Dispositif mélangeur (2) utilisable avec un appareil de mesure de débit (11) mélangeant ensemble au moins deux fluides médicaux délivrés à partir de tuyaux d'alimentation respectifs (6, 7), ledit dispositif mélangeur comprenant :
- un commutateur de pression (3) ayant une chambre de pression (11) de type membrane (12) montée dans le tuyau d'alimentation (6) d'un premier fluide,
- une soupape de commande de la pression (10), montée dans le tuyau d'alimentation de l'autre fluide, comprenant une chambre (15) ayant une entrée (16) et une sortie (17), un plongeur (18) et un clapet respectif (20) étant guidé vers et loin de ladite sortie (17) contre la sollicitation d'un moyen de ressort étalonné (19), ledit plongeur (18) étant raccordé à ladite membrane et commandé par la force de pression agissant sur ladite membrane (12), caractérisé en ce qu'il comprend,
- un injecteur calibré (26) raccordé au côté sortie de ladite chambre de pression (11) et au côté entrée d'un débitmètre (21) délivrant ledit premier fluide,
- une seconde chambre de pression (28) prévue avec une membrane élastique (29) et ayant une première entrée (37), raccordée au tuyau d'alimentation (6) dudit premier fluide de manière à contourner ledit commutateur de pression (3), et une seconde entrée (31) raccordée au côté sortie (14) dudit commutateur de pression (3) et séparée de la première entrée (37) par ladite membrane (29),
- ladite seconde chambre (28) ayant également une sortie (38) prévue sur le même côté que la seconde entrée (38) et raccordée au débitmètre (21) en aval dudit injecteur (26).

2. Dispositif mélangeur selon la revendication 1, caractérisé en ce qu'il comprend une autre conduite de dérivation (34) située entre le tuyau d'alimentation (6) et la première entrée (37) de la seconde chambre (28), ladite conduite (34) comportant un réservoir de stockage (35).

3. Dispositif mélangeur selon la revendication 2, caractérisé en ce qu'il comporte également un dispositif d'avertissement sonore (36) sur ladite conduite (34) en aval du réservoir (35) pour avertir de toute défaillance dans ladite pression du premier fluide.

4. Dispositif mélangeur selon la revendication 1, caractérisé en ce que ladite chambre de pression (28) est formée en un corps solide (32) recevant également la membrane élastique (29), un trou de passage (40) étant formé dans la partie solide du corps (32) procurant la première entrée (31) de ladite seconde chambre (28).

5. Dispositif mélangeur selon la revendication 1, caractérisé en ce que ladite soupape de commande de la pression (10) comporte une sortie (17) raccordée à une entrée respective d'un second débitmètre (23) par un injecteur calibré correspondant (27).
